# EUROPEAN PATENT SPECIFICATION

(11) **EP 1 784 174 B1**
(45) Date of publication and mention of the grant of the patent: **05.09.2012**
(21) Application number: 05774174.6
(22) Date of filing: 19.07.2005
(51) Int. Cl.: A61K 31/195, A61K 9/14, A61K 9/48

(54) **A PHARMACEUTICAL COMPOSITION COMPRISING GABAPENTIN**
PHARMAZEUTISCHE ZUSAMMENSETZUNG MIT GABAPENTIN
COMPOSITION PHARMACEUTIQUE COMPRENANT DE LA GABAPENTINE

(30) Priority: 20.07.2004 IT MI20041447
(43) Date of publication of application: 16.05.2007
(73) Proprietor: ZAMBON S.p.A., 20091 Bresso MI (IT)
(72) Inventor: RAMPOLDI, Luca, I-20020 LAINATE (IT); DE LAZZARI, Alessandra, I-35142 PADOVA (IT); GRASSANO, Alessandro, I-20052 MONZA (IT)
(86) International application number: PCT/EP2005/053473
(87) International publication number: WO 2006/008295

(56) References cited:
- WO-A-02/26263
- WO-A-2004/014356
- WO-A-2005/046566
- US-A- 6 054 482
- US-A1- 2004 010 035
- US-A1- 2004 092 522
- US-B1- 6 294 198

## Description

The present invention relates to pharmaceutical compositions. In particular, the invention relates to a pharmaceutical composition comprising gabapentin. Gabapentin is the common name of 1-aminomethyl-1-cyclohexan-acetic acid, a known drug with anti-epileptic activity, also effective in the treatment of neuropathic pain.

It is known that the formation of lactam with formula: 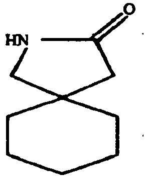 as a degradation product in the process for preparing and storing gabapentin is a drawback because of the toxicity of the lactam.

In the past, some attempts have been made to contrast the formation of the undesired lactamic byproduct and thus stabilise pharmaceutical compositions containing gabapentin.

The U.S.A. patent No. 6,054,482 in the name of Gödecke AG appears to solve the problem of favouring the conversion of less than 0.2% of gabapentin to the corresponding lactam, in a formulation whose initial lactam content is less than 0.5%, maintained for one year at 25°C and 60% relative humidity, thanks to a presence of anions of mineral acid below 20 ppm and of excipients that do not promote gabapentin dehydration. The same patent also lists a series of additives to be avoided in the composition because they promote the formation of lactam. They arc: modified maize starch, sodium croscarmelose, glycerol behenic acid ester, methacrylic acid co-polymers (type A and C), anion exchange resins, titanium dioxide, silica gel and PEG with low molecular weight.

On the other hand, U.S.A. patent No. 6,531,509 in the name of Tcva Pharmaceuticals Industries Ltd. states that the conditions supposed to assure stability, described in the aforementioned Gödecke AG patent, are technically unnecessary to achieve stability. According to the Tcva patent, not only stable gabapentin compositions can be obtained even when the mineral acid anion content exceeds 20 ppm, but in fact it is possible to use as preferred excipients those excipients that, according to the Gödecke patent, promoted the lactamic degradation of gabapentin.

The patent application No. WO 02/26263 by Sigmapharm describes stable gabapentin compositions containing a stabiliser that comprises a compound able to reduce ionic force, and at least 20 ppm of a mineral acid anion.

The stabilisers belongs to the following classes: volatile alcohols, non-volatile alcohols, non-volatile liquids, water miscible solids or liquids, water immiscible solids or liquids, liquid or solid surfactants, anti-oxidants, ketones or aldehydes.

It is readily apparent that not only the conflicting teachings of the aforementioned prior art are useless in solving the problem of lactam degradation in gabapentin formulations, but they are also likely to generate some confusion in a person skilled in the art.

Therefore, the ability to identify excipients suitable to control the formation of the lactamic impurity should be considered surprising in view both of the difficulties faced in the past in identifying suitable excipients, and of the confusion that actually can be noted in the state of the art with respect to excipient choice.

The present inventors met the need of identifying conditions that arc able to control gabapentin structural integrity without increasing its susceptibility to degradation to the corresponding lactamic form.

Therefore, it is a first object of the present invention a stable pharmaceutical composition comprising gabapentin as active ingredient, characterised in that it also comprises a mixture of excipients capable of not promoting the conversion of gabapentin into the corresponding lactamic impurity, which comprises:
(i) a sliding agent selected from a calcium salt of a weak acid,
(ii) a lubricating agent selected from hydrogenated castor oil and glyceryl behenate; and optionally
(iii) a diluting agent, alone or mixed with one or more other diluting agents, selected from a monosaccharidic sugar like sorbitol, xylitol, mannitol, fructose, dextrose and erythritol and polysaccharidic derivatives like saccharose, mannitol, isomalt, maltitol, a galactomannan, alginic acid or one of its salts, a pectin, a carrageenan and maltodextrin.

According to the present invention, a calcium salt of a weak acid is, preferably, tribasic calcium phosphate.

According to the present invention, a monosaccharidic sugar is, preferably, sorbitol. According to the present invention, a galactomannan is, preferably, guar rubber. According to the present invention a polysaccharide derivative is, preferably, alginic acid.

According to the present invention an alginic acid salt is sodium, potassium or calcium alginate, preferably sodium or calcium alginate, more preferably calcium alginate.

According to the present invention, a pectin is, preferably, citrus pectin.

According to the present invention a carrageenan is the kappa, iota, lambda or ksi carrageenan, or a calcium, ammonium or potassium salt thereof, preferably a calcium salt. In particular, a carrageenan is, preferably, the lambda carrageenan or a calcium salt thereof.

Moreover, the excipients listed above, when mixed with gabapentin even in small quantities with respect to the active principle, arc able to impart to the resulting mixture some favourable technological properties especially with regard to the sliding property.

This advantageous property can be exploited to facilitate pharmaceutical operations that entail allocating the powders, such as the manufacture of capsules and bags, without using complex technical approaches.

In a preferred aspect of the present invention, a diluting agent is present in a mixture with one or more diluting agents selected from those mentioned above. Preferably, the diluting agents in the mixture are sorbitol and alginic acid.

A particular pharmaceutical composition according to the invention comprises gabapentin as the active ingredient, tribasic calcium phosphate as a sliding agent, hydrogenated castor oil as a lubricant, sorbitol and alginic acid in mixture as diluents. In the pharmaceutical compositions according to the invention, the amount of gabapentin can vary from 50% to 99% of the total by weight of the composition, preferably from 70% to 98% of the total by weight of the composition; the amount of a sliding agent can vary from 0.5% to 5% of the total by weight of the composition, preferably from 1% to 3% of the total by weight of the composition; the amount of a lubricating agent can vary from 0.1% to 8% of the total by weight of the composition, preferably from 0.5% to 6% of the total by weight of the composition; and the amount of a diluting agent can vary from 0% to 50% of the total by weight of the composition, preferably from 0% to 40% of the total by weight of the composition.

The pharmaceutical compositions according to the present invention can be prepared according to the classic technique suggested by the state of the art, i.e. mixing the dry powders of each ingredient in a suitable mixer and the allocation of the mixture by means of a common encapsulating machine.

According to the present invention, a stable pharmaceutical composition containing gabapcntin is one in which the content of the corresponding lactamic impurity docs not exceed 0.2% by weight of gabapentin, after being maintained for 3 months at the storage conditions of 25°C with 60% of relative humidity, and/or at 30°C with 65% of relative humidity.

Some of the pharmaceutical compositions of the invention, evaluated as a representative example, have demonstrated that they meet the aforementioned conditions and they can therefore be considered stable for the purposes of the present invention.

Since, as stated above, the pharmaceutical compositions of the invention can be considered stable, not causing the undesired degradation of the active ingredient, they can be used successfully to prepare the pharmaceutical forms for oral use of gabapentin, in particular in the preparation of capsule, e.g. capsules made of hard gelatine or cellulose.

Therefore, a further object of the present invention is the use of the pharmaceutical composition as described above for the preparation of gabapentin capsules and the capsules that contain said composition.

The better to illustrate, without limiting, the present invention, the following examples are now provided.

### Example 1

### General procedure for the preparation of the pharmaceutical compositions according to the invention

In a Turbula Mod. T2F mixer of the company Bachofen (Basil - Switzerland) equipped with a metallic container with a capacity of 2 litres, a mixture of powders constituted by gabapcntin and by the appropriate excipients able not to promote the conversion of gabapentin into the corresponding lactamic impurity, is charged for 10 minutes at room temperature at a speed of 10 rpm.

The mixture of powders thus obtained, depending on the amount of ingredients introduced, can have a composition falling within the following values:
gabapentin 50-99% of the total by weight of the composition;
a sliding agent 0.5-5% of the total by weight of the composition;
a lubricating agent 0.1-8% of the total by weight of the composition; and
a diluting agent 0-50% of the total by weight of the composition.
The powders thus obtained are discharged and they are ready to be allocated in capsules.

### Example 2

With the procedure described in Example 1, the following pharmaceutical compositions were prepared. The quantities of the components being expressed in % of the component out of the total by weight of the composition.

### Composition 1

Gabapentin 75%
Sorbitol 15%
Alginic acid 4%
Hydrogenated castor oil 5%
Tribasic calcium phosphate 1%

### Composition 2

Gabapentin 70%
Fructose 12.5%
Pectin 10%
Carrageenan λ 5%
Glyceryl behenate 0.5%
Tribasic calcium phosphate 2%

### Composition 3

Gabapentin 90%
Mannitol 3%
Calcium alginate 1%
Hydrogenated castor oil 3%
Tribasic calcium phosphate 3%

### Composition 4

Gabapentin 95%
Tribasic calcium phosphate 4%
Glyceryl behenate 1%

### Composition 5

Gabapentin 80%
Xylitol 10%
Erythritol 1%
Maltodextrin 5%
Glyceryl behenate 2%
Tribasic calcium phosphate 2%

### Composition 6

Gabapentin 60%
Isomalt 10%
Saccharose 20%
Alginic acid 9%
Hydrogenated castor oil 0.5%
Tribasic calcium phosphate 0.5%

### Composition 7

Gabapentin 75%
Dextrose 10%
Guar rubber 7%
Hydrogenated castor oil 6%
Tribasic calcium phosphate 2%

### Composition 8

Gabapentin 85%
Maltitol 5 %
Tribasic calcium phosphate 2%
Glyceryl behenate 1%
Hydrogenated castor oil 7%

### Composition 9

Gabapentin 90%
Sorbitol 3%
Sodium alginate 1%
Glyceryl behenate 2%
Hydrogenated castor oil 1%
Tribasic calcium phosphate 3%

### Example 3

The pharmaceutical compositions of Example 2 were used separately to fill capsules made of hard gelatine and cellulose, obtaining pharmaceutical forms of gabapentin in capsules, by using an IN-CAP Model automatic encapsulating machine by the company Dott. Bonapace (Milan-Italy).

## Claims

1. A stable pharmaceutical composition comprising gabapentin as active ingredient, **characterised in that** it also comprises a mixture of excipients capable of not promoting the conversion of gabapentin into the corresponding lactamic impurity, which comprises:
(i) a sliding agent selected from a calcium salt of a weak acid,
(ii) a lubricating agent selected from hydrogenated castor oil and glyceryl behenate; and optionally
(iii) a diluting agent, alone or mixed with one or more other diluting agents, selected from a monosaccharidic sugar like sorbitol, xylitol, mannitol, fructose, dextrose and erythritol and polysaccharidic derivatives like saccharose, mannitol, isomalt, maltitol, a galactomannan, alginic acid or one of its salts, a pectin, a carrageenan and maltodextrin.

2. A composition as claimed in claim 1, wherein gabapentin is present in amount variable between 50% and 99% of the total by weight of the composition.

3. A composition as claimed in claim 1, wherein a sliding agent is present in amount variable between 0.5% and 5% of the total by weight of the composition.

4. A composition as claimed in claim 1, wherein a lubricating agent is present in amount variable between 0.1% and 8% of the total by weight of the composition.

5. A composition as claimed in claim 1, wherein a diluting agent is present in amount variable between 0% and 50% of the total by weight of the composition.

6. A composition as claimed in claim 1, wherein a calcium salt of a weak acid is tribasic calcium phosphate.

7. A composition as claimed in claim 1, wherein a diluting agent is present in a mixture with one or more diluting agents selected from those described in claim 1.

8. A composition as claimed in claim 7, wherein the diluting agents in the mixture arc sorbitol and alginic acid.

9. A composition as claimed in claim 1, wherein a galactomannan is guar rubber.

10. A composition as claimed in claim 1, wherein a salt of alginic acid is sodium, potassium or calcium alginate.

11. A composition as claimed in claim 1, wherein a pectin is citrus pectin.

12. A composition as claimed in claim 1, wherein a carrageenan is the kappa, iota, lambda or ksi carrageenan, or a calcium, ammonium or potassium salt thereof.

13. A pharmaceutical composition as claimed in claim 1, wherein the calcium salt of a weak acid is tribasic calcium phosphate, the lubricant is hydrogenated castor oil, and sorbitol as a diluting agent is present in a mixture with another diluting agent, which is alginic acid.

14. Use of a pharmaceutical composition as claimed in claim 1, for the preparation of capsules.

15. Capsules containing a pharmaceutical composition as claimed in claim 1.

## Patentansprüche

1. Stabile pharmazeutische Zusammensetzung, welche Gabapentin als aktiven Inhaltsstoff umfasst, **dadurch gekennzeichnet, dass** sie auch ein Gemisch von Trägerstoffen umfasst, die in der Lage sind, nicht die Umwandlung von Gabapentin in die entsprechende Lactam-Verunreinigung zu fördern, wobei das Gemisch das Folgende umfasst:
(i) ein Gleitmittel, welches aus Calciumsalzen schwacher Säuren ausgewählt ist;
(ii) ein Schmiermittel, welches aus hydriertem Rizinusöl und Glycerylbehenat ausgewählt ist; und gegebenenfalls
(iii) ein Verdünnungsmittel, allein oder mit einem oder mehreren anderen Verdünnungsmitteln vermischt, die aus einem Monosaccharid-Zucker wie Sorbitol, Xylitol, Mannitol, Fructose, Dextrose und Erythritol und Polysaccharid-Derivaten wie Saccharose, Mannitol, Isomalt, Maltitol, einem Galactomannan, Alginsäure oder einem ihrer Salze, einem Pektin, einem Carrageenan und Maltodextrin ausgewählt sind.

2. Zusammensetzung nach Anspruch 1, wobei das Gabapentin in einer variablen Menge von 50 % bis 99 % des Gesamtgewichts der Zusammensetzung vorliegt.

3. Zusammensetzung nach Anspruch 1, wobei das Gleitmittel in einer variablen Menge von 0,5 % bis 5 % des Gesamtgewichts der Zusammensetzung vorliegt.

4. Zusammensetzung nach Anspruch 1, wobei das Schmiermittel in einer variablen Menge von 0,1 % bis 8 % des Gesamtgewichts der Zusammensetzung vorliegt.

5. Zusammensetzung nach Anspruch 1, wobei das Verdünnungsmittel in einer variablen Menge von 0 % bis 50 % des Gesamtgewichts der Zusammensetzung vorliegt.

6. Zusammensetzung nach Anspruch 1, wobei es sich bei dem Calciumsalz einer schwachen Säure um dreibasiges Calciumphosphat handelt.

7. Zusammensetzung nach Anspruch 1, wobei ein Verdünnungsmittel in einem Gemisch mit einem oder mehreren Verdünnungsmitteln vorliegt, die aus jenen in Anspruch 1 angegebenen ausgewählt sind.

8. Zusammensetzung nach Anspruch 7, wobei es sich bei den Verdünnungsmitteln in dem Gemisch um Sorbitol und Alginsäure handelt.

9. Zusammensetzung nach Anspruch 1, wobei es sich bei dem Galactomannan um Guaran handelt.

10. Zusammensetzung nach Anspruch 1, wobei es sich bei dem Salz der Alginsäure um Natrium-, Kalium- oder Calciumalginat handelt.

11. Zusammensetzung nach Anspruch 1, wobei es sich bei dem Pektin um Citruspektin handelt.

12. Zusammensetzung nach Anspruch 1, wobei es sich bei dem Carrageenan um das Kappa-, Iota-, Lambda- oder Xi-Carrageenan oder ein Calcium-, Ammonium- oder Kaliumsalz derselben handelt.

13. Pharmazeutische Zusammensetzung nach Anspruch 1, wobei es sich bei dem Calciumsalz einer schwachen Säure um dreibasiges Calciumphosphat handelt, bei dem Schmiermittel um hydriertes Rizinusöl handelt und Sorbitol als Verdünnungsmittel in einem Gemisch mit einem anderen Verdünnungsmittel vorliegt, bei welchem es sich um Alginsäure handelt.

14. Verwendung einer pharmazeutischen Zusammensetzung nach Anspruch 1 zur Herstellung von Kapseln.

15. Kapseln, welche eine pharmazeutische Zusammensetzung nach Anspruch 1 enthalten.

## Revendications

1. Composition pharmaceutique stable qui comprend de la gabapentine en tant que principe actif, **caractérisée en ce qu'**elle comprend également un mélange d'excipients capables de ne pas promouvoir la conversion de la gabapentine en l'impureté lactamique correspondante, qui comprend :
(i) un agent de glissement choisi parmi un sel de calcium d'un acide faible,
(ii) un agent lubrifiant choisi parmi l'huile de ricin hydrogéné et le béhénate de glycéryle ; et facultativement
(iii) un agent de dilution, seul ou mélangé avec un ou plusieurs autres agents de dilution, choisis parmi un sucre monosaccharidique tel que le sorbitol, le xylitol, le mannitol, le fructose, le dextrose et l'érythritol et des dérivés polysaccharidiques tels que le saccharose, le mannitol, l'isomalt, le maltitol, un galactomannane, l'acide alginique ou l'un de ses sels, une pectine, une carraghénine et une maltodextrine.

2. Composition selon la revendication 1, dans laquelle la gabapentine est présente en une quantité variant de 50% à 99% du total en poids de la composition.

3. Composition selon la revendication 1, dans laquelle un agent de glissement est présent en une quantité variant de 0,5% à 5% du total en poids de la composition.

4. Composition selon la revendication 1, dans laquelle un agent lubrifiant est présent en une quantité variant de 0,1% à 8% du total en poids de la composition.

5. Composition selon la revendication 1, dans laquelle un agent de dilution est présent en une quantité variant de 0% à 50% du total en poids de la composition.

6. Composition selon la revendication 1, dans laquelle un sel de calcium d'un acide faible est un phosphate de calcium tribasique.

7. Composition selon la revendication 1, dans laquelle un agent de dilution est présent dans un mélange avec un ou plusieurs agents de dilution choisis parmi ceux décrits dans la revendication 1.

8. Composition selon la revendication 7, dans laquelle les agents de dilution dans le mélange sont le sorbitol et l'acide alginique.

9. Composition selon la revendication 1, dans laquelle un galactomannane est la gomme de guar.

10. Composition selon la revendication 1, dans laquelle un sel de l'acide alginique est l'alginate de sodium, de potassium ou de calcium.

11. Composition selon la revendication 1, dans laquelle une pectine est la pectine d'agrumes.

12. Composition selon la revendication 1, dans laquelle une carraghénine est la kappa, iota, lambda ou ksi carraghénine, ou un sel de calcium, d'ammonium ou de potassium de celle-ci.

13. Composition pharmaceutique selon la revendication 1, dans laquelle le sel de calcium d'un acide faible est le phosphate de calcium tribasique, le lubrifiant est de l'huile de ricin hydrogéné, et du sorbitol en tant qu'agent de dilution est présent dans un mélange avec un autre agent de dilution, qui est l'acide alginique.

14. Utilisation d'une composition pharmaceutique selon la revendication 1, pour la préparation de gélules.

15. Gélules contenant une composition pharmaceutique selon la revendication 1.
